# FASCICULE DE BREVET EUROPEEN

(11) **EP 0 752 988 B1**
(45) Date de publication et mention de la délivrance du brevet: **26.05.1999**
(21) Numéro de dépôt: 95914405.6
(22) Date de dépôt: 23.03.1995
(51) Int. Cl.: C07D 241/38, C07D 487/04, A61K 31/495

(54) **DERIVES DE 5H-INDENO[1,2-b]PYRAZINE-2,3-DIONE ANTAGONISTES DES RECEPTEURS AMPA ET NMDA**
5H-INDENO[1,2-b]PYRAZIN-2,3-DION-DERIVATE ANTAGONISTEN DER AMPA UND NMDA REZEPTOREN
5H-INDENO[1,2-b]PYRAZINE-2,3-DIONE DERIVATIVES ANTAGONISTS OF AMPA AND NMDA RECEPTORS

(30) Priorité: 28.03.1994 FR 9403583
(43) Date de publication de la demande: 15.01.1997
(73) Titulaire: RHONE-POULENC RORER S.A., 92160 Antony (FR)
(72) Inventeur: ALOUP, Jean-Claude, F-94290 Villeneuve-le-Roi (FR); AUDIAU, François, F-94220 Charenton-le-Pont (FR); BARREAU, Michel, F-91230 Montgeron (FR); DAMOUR, Dominique, F-75014 Paris (FR); GENEVOIS-BORELLA, Arielle, F-94320 Thiais (FR); JIMONET, Patrick, F-78450 Villepreux (FR); MAGNANI, Serge, F-92290 Châtenay-Malabry (FR); RIBEILL, Yves, F-91360 Villemoisson-sur-Orge (FR)
(74) Mandataire: Savina, Jacques
(86) Numéro de dépôt international: FR9500359
(87) Numéro de publication internationale: WO9526342

(56) Documents cités:
- WO-A-94/00124
- WO-A-94/18175

## Description

La présente invention concerne des composés de formule : 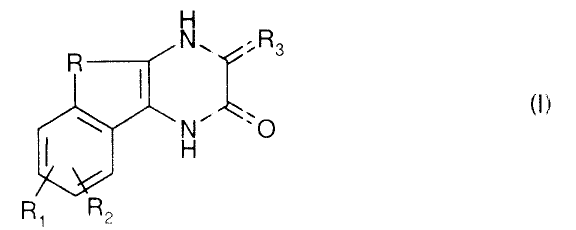 leurs sels, leur préparation et les médicaments les contenant.

Des antagonistes NMDA dérives de 1,4-dihydroquinoxaline-2,3-dione sont décrits dans la demande de brevet WO94/00124. Par ailleurs, des dérivés de 9H-indéno[1,2-b]pyrazine-2,3(1H, 4H)-dione sont décrits dans la demande de brevet WO94/18175 publiée le 18 Août 1994.

Dans la formule (I),
- R représente un radical C(R₄)R₅, CH-R₆ ou C=R₇,
- R₁ et R₂, identiques ou différents, représentent des atomes d'hydrogène ou d'halogène ou des radicaux amino, nitro ou -NH-CO-NR₁₁R₁₂,
- R₃ représente un atome d'oxygène,
- R₄ représente un radical alkyle,
- R₅ représente un radical -alk-COOR₁₀,
- R₆ représente un atome d'hydrogène ou un radical -NR₁₄R₁₅,
- R₇ représente un radical NOH ou C(COOR₁₀)R₂₀,
- R₁₀ représente un atome d'hydrogène ou un radical alkyle,
- R₁₁ représente phényle,
- R₁₂ représente un atome d'hydrogène,
- R₁₄ représente un atome d'hydrogène,
- R₁₅ représente un atome d'hydrogène ou un radical -COR₂₂,
- R₂₀ représente un atome d'hydrogène,
- R₂₂ représente un radical alkyle,
- alk représente un radical alkyle,
étant entendu que lorsque R₁ et R₂ représentent des atomes d'hydrogène et R₃ représente un atome d'oxygène, R n'est pas un radical C=R₇ dans lequel R₇ représente un radical NOH.

Sauf mention contraire, dans les définitions qui précédent et celles qui suivent, les radicaux et portions alkyle contiennent 1 à 6 atomes de carbone et sont en chaîne droite ou ramifiée et les atomes d'halogène sont choisis parmi le fluor, le chlore, le brome et l'iode.

Les composés de formule (I) pour lesquels R représente un radical C=R₇ pour lequel R₇ représente un radical C(COOR₁₀)R₂₀ présentent des formes isomères (E et Z). Ces isomères et leurs mélanges font partie de l'invention.

Les énantiomères et diastéréoisomères des composés de formule (I) pour lesquels R représente un radical C(R₄)R₅ ou CH-R₆ font également partie de l'invention.

Les composés de formule (I) pour lesquels R représente un radical C(R₄)R₅ ou CH-R₆, R₆ représente un atome d'hydrogène et R₃ représente un atome d'oxygène peuvent être préparés par cyclisation en présence d'acétate d'ammonium d'un dérivé de formule : 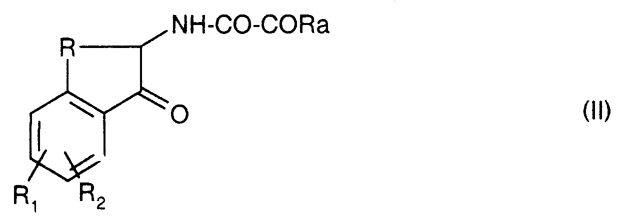 dans laquelle R représente un radical C(R₄)R₅ ou CH-R₆ dans lequel R₆ représente un atome d'hydrogène, R₁ et R₂ ont les mêmes significations que dans la formule (I) et Ra représente un radical alcoxy.

Cette réaction s'effectue, de préférence au sein de l'acide acétique, à la température d'ébullition du milieu réactionnel.

Les dérivés de formule (II) peuvent être obtenus par action d'un chlorure ClCOCORa dans lequel Ra est un radical alcoxy sur un dérivé de formule : 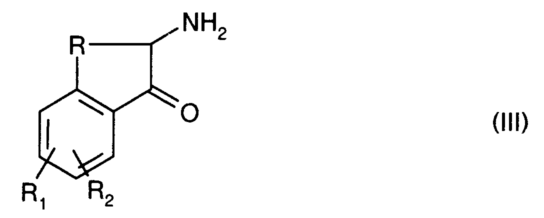 dans laquelle R représente un radical C(R₄)R₅ ou CH-R₆ dans lequel R₆ représente un atome d'hydrogène, R₁ et R₂ ont les mêmes significations que dans la formule (I).

Cette réaction s'effectue généralement au sein d'un solvant inerte tel que le tétrahydrofuranne, en présence d'une amine tertiaire telle que la triéthylamine, à une température comprise entre 0 et 25°C

Les dérivés de formule (III) peuvent être obtenus par application ou adaptation des méthodes décrites dans les exemples et par P.W. NEBER et coll., Justus Liebigs Ann. Chem., 526, 277 (1936), V.S. VELEZHEVA et coll., Khim. Farm. Zh., 24, 46 (1990) (Chem. Abstracts, 114, 228786), YUHPYNG L. CHEN et coll., J. Med. Chem., 35(8), 1429 (1992).

Les composés de formule (I) pour lesquels R représente un radical C=R₇ et R₇ représente un radical NOH peuvent être préparés par action d'un nitrite d'alkyle sur un composé de formule (I) correspondant pour lequel R représente un radical CH-R₆ et R₆ représente un atome d'hydrogène.

Cette réaction s'effectue de préférence au sein d'un solvant inerte tel que le diméthylsulfoxyde, en présence d'un hydrure de métal alcalin tel que l'hydrure de sodium, à une température voisine de 20°C. De préférence, on utilise le nitrite d'isoamyle.

Les composés de formule (I) pour lesquels R représente un radical C=R₇ et R₇ représente un radical C(COOR₁₀)R₂₀ peuvent être préparés par déshydratation d'un dérivé de formule : 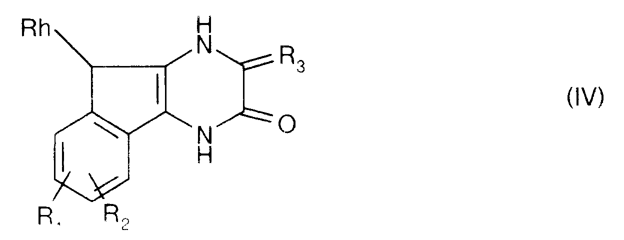 dans laquelle R₁, R₂ et R₃ ont les mêmes significations que dans la formule (I) et Rh représente un radical -C(R₂₀)(OH)-COOR₁₀ dans lequel R₂₀ et R₁₀ ont les mêmes significations que dans la formule (I).

Cette réaction s'effectue généralement au sein de l'anhydride acétique, à la température d'ébullition du milieu réactionnel.

Les dérivés de formule (IV) pour lesquels R₁, R₂ et R₃ ont les mêmes significations que dans la formule (I) et Rh représente un radical -C(R₂₀)(OH)-COOR₁₀ dans lequel R₂₀ et R₁₀ ont les mêmes significations que dans la formule (I) peuvent être obtenus par action d'un composé de formule (I) correspondant pour lequel R représente un radical CH-R₆ et R₆ représente un atome d'hydrogène sur un dérivé R₂₀-CO-COOR₁₀ pour lequel R₁₀ et R₂₀ ont les mêmes significations que dans la formule (I), suivie d'un traitement à l'acide acétique.

Cette réaction s'effectue au sein du diméthylsulfoxyde, en présence d'un hydrure de métal alcalin tel que l'hydrure de sodium, à une temperature voisine de 20°C ou en présence de bromure de tétrabutylammonium et d'une base telle qu'un hydroxyde de métal alcalin (soude, potasse par exemple), au sein du diméthylsulfoxyde, à une température comprise entre 20 et 100°C. Le traitement à l'acide acétique s'effectue à une température inférieure à 20°C.

Les dérivés de formule R₂₀-CO-COOR₁₀ dans laquelle R₁₀ et R₂₀ ont les mêmes significations que dans la formule (I) sont commercialisés ou peuvent être obtenus par application ou adaptation des méthodes décrites par L.A. CARPINO, J. Org. Chem., 29, 2820 (1964) et H.H. WASSERMAN, J. Org. Chem., 50, 3573 (1985).

Les composés de formule (I) pour lesquels R représente un radical CH-R₆ dans lequel R₆ représente un radical -NR₁₄R₁₅, R₁₄ et R₁₅ représentent chacun un atome d'hydrogène peuvent être préparés par hydrolyse d'un composé de formule (I) correspondant pour lequel R représente un radical CH-R₆ dans lequel R₆ représente un radical -NR₁₄R₁₅, R₁₄ représente un atome d'hydrogène et R₁₅ représente un radical -COR₂₂ et R₂₂ représente un radical alkyle.

Cette hydrolyse s'effectue généralement au moyen d'un acide tel que l'acide chlorhydrique, en milieu aqueux, à la température d'ébullition du milieu réactionnel.

Les composés de formule (I) pour lesquels R représente un radical CH-R₆ dans lequel R₆ représente un radical -NR₁₄R₁₅, R₁₄ représente un atome d'hydrogène et R₁₅ représente un radical -COR₂₂ et R₂₂ représente un radical alkyle peuvent être préparés par action d'un agent réducteur sur un composé de formule (I) correspondant pour lequel R représente un radical C=R₇ et R₇ représente un radical NOH, puis d'un traitement avec un anhydride (RqCO)₂O pour lequel Rq représente un radical alkyle.

Cette réaction s'effectue généralement à une température comprise entre 50 et 100°C. Comme agent réducteur on utilise de préférence le zinc.

Les composés de formule (I) pour lesquels R₁ et éventuellement R₂ représentent un radical -NH-CO-NR₁₁R₁₂ et R₁₂ représente un atome d'hydrogène peuvent également être préparés par action d'un composé de formule (I) correspondant pour lequel R₁ représente un radical amino sur un dérivé Rx=C=N-Ry dans lequel Rx représente un atome d'oxygène et Ry représente un radical phényle.

Cette réaction s'effectue généralement au sein d'un solvant inerte tel que le diméthylformamide, le tétrahydrofuranne ou le dioxanne, à une température comprise entre 20°C et la température d'ébullition du milieu réactionnel suivie d'une hydrolyse du dérivé silylé pour obtenir le composé pour lequel R₁₂ représente un atome d'hydrogène, au moyen d'une solution aqueuse, à une température comprise entre 20 et 50°C.

Il est entendu pour l'homme du métier que, pour la mise en oeuvre des procédés selon l'invention décrits précédemment, il peut être nécessaire d'introduire des groupes protecteurs des fonctions amino, hydroxy et carboxy afin d'éviter des réactions secondaires. Ces groupes sont ceux qui permettent d'être éliminés sans toucher au reste de la molécule. Comme exemples de groupes protecteurs de la fonction amino on peut citer les carbamates de tert-butyle ou de méthyle qui peuvent être régénérées au moyen d'iodotriméthylsilane. Comme exemples de groupes protecteurs de la fonction hydroxy, on peut citer les triéthylsilyle, benzyle. Comme groupes protecteurs des fonctions carboxy, on peut citer les esters (méthoxyméthylester, tétrahydropyranylester, benzylester par exemple, les oxazoles et les 2-alkyl-1,3-oxazolines. D'autres groupes protecteurs utilisables sont décrits par W. GREENE et coll., Protecting Groups in Organic Synthesis, second edition, 1991, Jonh Wiley & Sons.

Les composés de formule (I) peuvent être purifiés par les méthodes connues habituelles, par exemple par cristallisation, chromatographie ou extraction.

Les énantiomères des composés de formule (I) pour lesquels R représente un radical C(R₄)R₅ ou CH-R₆ peuvent être obtenus par dédoublement des racémiques par exemple par chromatographie sur colonne chirale selon W.H. PIRCKLE et coll., asymetric synthesis, vol. 1. Academic Press (1983) ou par synthèse à partir des précurseurs chiraux.

Les diastéréoisomères des composes de formule (I) pour lesquels R représente un radical C(R₄)R₅ ou CH-R₆ comportant un ou plusieurs carbones chiraux et les différents isomères E et Z des composés de formule (I) peuvent être séparés par les méthodes connues habituelles, par exemple par cristallisation ou chromatographie.

Les composés de formule (I) comportant un reste basique peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) comportant un reste acide peuvent éventuellement être transformés en sels métalliques ou en sels d'addition avec les bases azotées selon des méthodes connues en soi. Ces sels peuvent être obtenus par action d'une base métallique (alcaline ou alcalinoterreuse par exemple), de l'ammoniac, d'une amine ou d'un sel d'une amine sur un composé de formule (I), dans un solvant. Le sel formé est séparé par les méthodes habituelles.

Ces sels font également partie de l'invention.

Comme exemples de sels pharmaceutiquement acceptables, peuvent être cités les sels d'addition avec les acides minéraux ou organiques (tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate), les sels avec les métaux alcalins (sodium, potassium, lithium) ou avec les métaux alcalinoterreux (calcium, magnésium), le sel d'ammonium, les sels de bases azotées (éthanolamine, triméthylamine, méthylamine, benzylamine, N-benzyl-β-phénéthylamine, choline, arginine, leucine, lysine, N-méthyl glucamine).

Les composés de formule (I) présentent des propriétés pharmacologiques intéressantes. Ces composés sont des antagonistes du récepteur de l'acide α-amino-3-hydroxy-5-méthyl-4-isoxazolepropionique (AMPA), connu aussi sous le nom de récepteur du quisqualate.

Par ailleurs, les composés de formule (I) sont des antagonistes non compétitifs du récepteur N-méthyl-D-aspartate (NMDA) et, plus particulièrement, ce sont des ligands pour les sites modulateurs de la glycine du récepteur NMDA.

Ces composés sont donc utiles pour traiter ou prévenir toutes les ischémies (telles l'ischémie focale ou globale) consécutives à des accidents vasculaires cérébraux, un arrêt cardiaque, une hypotension artérielle, une intervention chirurgicale cardiaque ou pulmonaire ou une hypoglycémie sévére. Ils sont également utiles dans le traitement des effets dus à une anoxie, qu'elle soit périnatale ou consécutive à une noyade ou à des lésions cérébro-spinales. Ces composés peuvent également être utilisés pour traiter ou prévenir l'évolution de maladies neurodégénératives, de la chorée d'HUNTINGTON, de la maladie d'ALZHEIMER, de la sclérose latérale amyotrophique, de l'atrophie olivo-pontocérébelleuse et de la maladie de PARKINSON. Ces composés peuvent aussi être utilisés vis-à-vis des manifestations épileptogènes et/ou convulsives, pour le traitement des traumatismes cérébraux ou spinaux, des traumatismes liés à la dégénérescence de l'oreille interne (R. PUJOL et coll., Neuroreport, 3, 299-302 (1992) ou de la rétine (J.L. MONSINGER et coll., Exp. Neurol., 113, 10-17 (1991), de l'anxiété (KEHNE et coll., Eur. J. Pharmacol., 193, 283 (1991)), de la dépression (TRULLAS et coll.,Eur. J. Pharmacol., 185, 1 (1990)), de la schizophrénie (REYNOLDS, TIPS, 13, 116 (1992)), du syndrome de TOURETTE, des encéphalopathies hépatiques, en tant qu'analgésiques (DICKENSON et coll., Neurosc. Letters, 121, 263 (1991)), antiinflammatoire (SLUTA et coll., Neurosci. Letters, 149, 99-102 (1993)) antianorexiques (SORRELS et coll., Brain Res., 572, 265 (1992)), antimigraineux, antiémétiques et pour traiter les empoisonnements par des neurotoxines ou d'autres substances agonistes du récepteur NMDA, ainsi que les troubles neurologiques associés aux maladies virales telles que le sida (LIPTON et coll., Neuron, 7, 111 (1991)), la rage, la rougeole et le tétanos (BAGETTA et coll., Br. J. Pharmacol., 101, 776 (1990)). Ces composés sont aussi utiles pour la prévention des symptômes d'abstinence aux drogues et à l'alcool et de l'inhibition de l'accoutumance et de la dépendance aux opiacés. Ils peuvent également être utilisés dans le traitement des déficits liés à des anomalies mitochondriales telles que la myopathie mitochondriale, le syndrome de LEBER, l'encéphalopathie de WERNICKE, le syndrome de RETT, l'homocystéinémie, l'hyperprolinémie, l'hydroxybutirique-aminoacidurie, l'encéphalopathie saturnine (intoxication chronique au plomb) et la déficience en sulfite oxydase.

L'affinité des composés de formule (I) vis-à-vis du récepteur AMPA a été déterminée en étudiant l'antagonisme de la fixation spécifique du [³H]-AMPA sur des membranes de cortex cérébral de rat (HONORE et coll., Neuroscience letters, 54, 27 (1985)). Le [³H]-AMPA est mis à incuber en présence de 0,2 mg de protéines à 4°C pendant 30 minutes dans du tampon KH₂PO₄ 10mM, KSCN 100mM, pH7,5. La fixation non spécifique est déterminée en présence de L-glutamate 1mM. La radioactivité liée est séparée par filtration sur filtres PHARMACIA (Printed Filtermate A). L'activité inhibitrice de ces produits est inférieure ou égale à 100 µM.

L'affinité des composés de formule (I) pour le site glycine lié au récepteur NMDA a été déterminée en étudiant l'antagonisme de la fixation spécifique du [³H]-DCKA sur des membranes de cortex cérébral de rat selon la méthode décrite par T. CANTON et coll., J. Pharm. Pharmacol., 44, 812 (1992). Le [³H]-DCKA (20nM) est mis à incuber en présence de 0,1 mg de protéines à 4°C pendant 30 minutes dans du tampon HEPES 50 mM, pH7,5. La fixation non spécifique est déterminée en présence de glycine 1mM. La radioactivité liée est séparée par filtration sur filtres Whatman GF/B. L'activité inhibitrice de ces produits est inférieure ou égale à 100 µM.

Les composés de formule (I) présentent une toxicité faible. Leur DL50 est supérieure à 50 mg/kg par voie IP chez la souris.

Les composés préférés sont les suivants :
- 1,4-dihydro-5H-indéno[1,2-b]pyrazine-2,3-dione,
- 8-chloro-1,4-dihydro-5H-indeno[1,2-b]pyrazine-2,3-dione,
- acide (5-méthyl-2,3-dioxo-1,4-dihydro-5H-indéno[1,2-b]pyrazine-5-yl)acétique,
- 5-amino-8-chloro-1,4-dihydro-5H-indéno[1,2-b]pyrazine-2,3-dione,
- 7-(3-phényluréido)-1,4-dihydro-5H-indéno[1,2-b]pyrazine-2,3-dione,
- (E)-5-carboxyméthylène-8-chloro-1,4-dihydro-indéno[1,2-b]pyrazine-2,3-dione,
- acide (8-chloro-5-méthyl-2,3-dioxo-1,4-dihydro-5H-indéno[1,2-b]pyrazine-5-yl)acétique,
- (+) acide (5-méthyl-2,3-dioxo-1,4-dihydro-1H-indéno[1,2-b]pyrazine-5-yl)acétique,
- (-) acide (5-méthyl-2,3-dioxo-1,4-dihydro-1H-indéno[1,2-b]pyrazine-5-yl)acétique,
- (+) acide (8-chloro-5-méthyl-2,3-dioxo-1,4-dihydro-5H-indéno[1,2-b]pyrazine-5-yl)acétique,
- (-) acide (8-chloro-5-méthyl-2,3-dioxo-1,4-dihydro-5H-indéno[1,2-b]pyrazine-5-yl)acétique
   et leurs sels.

Les exemples suivants illustrent l'invention.

### EXEMPLE 1

A une solution de 12,5 g d'acétate d'ammonium dans 50 ml d'acide acétique portés à reflux sous azote, on ajoute rapidement 1,25 g de 2-éthoxalylamino-1-indanone. Après 18 heures de reflux, le milieu réactionnel est refroidi jusqu'à une température voisine de 20°C. Le précipité formé est filtré, lavé abondemment à l'eau et séché sous vide partiel (1 mm Hg; 0,13 kPa) à 50°C. 0,77 g de 1,4-dihydro-5H-indéno[1,2-b]pyrazine-2,3-dione sont ainsi obtenus sous forme de poudre verte dont le point de fusion est supérieur à 260°C (Analyse C11H8N2O2; 1,18H2O % calculé C : 66,00; H : 4,03; N : 13,99; % trouvé C : 65,7; H :3,9; N :13,9).

La 2-éthoxalylamino-1-indanone peut être préparée selon le protocole suivant : 6 g du chlorhydrate de 2-amino-1-indanone et 26,7 ml de chlorure d'éthyloxalate sont dissous dans 300 ml de tétrahydrofuranne et agités sous azote. 26,7 ml de triéthylamine sont ajoutés goutte à goutte au mélange réactionnel et la réaction poursuivie 90 minutes à une température voisine de 20°C. Le mélange est alors filtre sur célite et le filtrat concentré à sec sous pression réduite (15 mm Hg; 2 kPa). Le résidu est purifié par flashchromatographie sur colonne de silice en utilisant un mélange d'acétate d'éthyle et de cyclohexane (20/80 en volumes) comme éluant. 1,5 g de produit attendu sont ainsi obtenus sous forme de solide orangé fondant à 135°C.

Le chlorhydrate de 2-amino-1-indanone peut être obtenu de la manière suivante : une solution d'éthylate de sodium, préparée à partir de 15,2 g de sodium et 770 ml d'éthanol absolu, est ajoutée goutte à goutte, à 0°C et sous azote, à 200 g de p-toluènesulfonate de 1-indanone-oxime en solution dans 3,7 litres de toluène anhydre. La réaction est poursuivie 40 heures à 0°C. La suspension est alors filtrée sur célite et le filtrat lavé à l'eau (400 ml). La solution toluènique est traitée par de l'acide chlorhydrique 1N (4 x 400 ml) et la phase aqueuse concentrée à sec sous presssion réduite (15 mm Hg; 2 kPa) à 40°C. Le résidu obtenu est repris dans l'acétone, filtré et séché pour conduire à 29,8 g de produit attendu sous forme de solide brun qui se décompose par chauffage.

Le p-toluènesulfonate de 1-indanone-oxime peut être préparé de la façon suivante : 532 g de chlorure de p-toluènesulfonate dissous dans 600 ml de pyridine sont ajoutés goutte à goutte à 186,1 g de 1-indanone-oxime en solution dans 900 ml de pyridine à 0°C et sous azote. Après 3 heures de réaction à 0°C, le milieu réactionnel est versé dans 3 litres d'eau glacée. Le précipité formé est filtré, lavé à l'eau et séché. On obtient ainsi 354 g de produit attendu sous forme de solide blanc fondant à 150°C.

La 1-indanone-oxime peut être obtenue de la manière suivante : à 200 g de 1-indanone en solution dans 3,4 litres de méthanol, on ajoute 366 g de chlorhydrate d'hydroxylamine, 366 g de carbonate de potassium et 340 ml d'eau distillée. Le mélange est porté a reflux pendant 18 heures. Après refroidissement, la suspension formée est filtrée, lavée à l'eau puis au méthanol et séchée sous presssion réduite (1 mm Hg; 0,13 kPa) pour conduire à 186,1 g de produit attendu sous forme de solide blanc fondant à 154°C.

### EXEMPLE 2

35 g d'acétate d'ammonium sont solubilisés dans 70 ml d'acide acétique. A cette solution, on ajoute 12,2 g de 6-chloro-2-éthoxalylamino-1-indanone et on porte le mélange à reflux pendant 6 heures. Le milieu réactionnel est alors concentré à sec sous presssion reduite (15 mm Hg; 2 kPa) et l'huile ainsi obtenue reprise avec 100 ml d'eau distillée. Le précipité formé est filtré, lavé à l'eau et séché sous vide partiel (1 mm Hg; 0,13 kPa) à 40°C. On obtient 8,05 g de 8-chloro-1,4-dihydro-5H-indeno[1,2-b]pyrazine-2,3-dione sous forme de solide brun dont le point de fusion est supérieur à 260°C (Spectre de R.M.N.: [300 MHz; (CD₃)₂SO d6; δ en ppm] : 3,56 (s, 2H : -CH₂- 9); 7,15 (dd, J = 8 et 2 Hz, 1H : -H 7); 7,43 (d, J = 8 Hz, 1H : -H 8); 7,62 (d, J = 2 Hz, 1H : -H 5); de 11,75 à 12,75 (mf très étalé: -NHCO-)).

La 6-chloro-2-éthoxalylamino-1-indanone peut être obtenue de la manière suivante : dans un tricol refroidi à 0°C, on met en suspension 8,4 g de chlorhydrate de 2-amino-6-chloro-1-indanone dans 100 ml de tétrahydrofuranne, puis on ajoute 5,53 ml de chlorure d'éthyloxalate en solution dans 30 ml du même solvant et enfin, goutte à goutte de manière à ce que la température ne s'élève pas, 12,7 ml de triéthylamine en solution dans 70 ml de tétrahydrofuranne. La réaction est poursuivie 2 heures pendant lesquelles la température remonte lentement jusqu'à 20°C. Le précipité formé est filtré sur célite et le filtrat concentré à sec sous pression réduite. Le résidu obtenu est repris dans 50 ml d'éther éthylique, filtré et séché sous vide partiel (1 mm Hg; 0,13 kPa) à 40°C. On obtient ainsi 9,3 g de produit attendu sous forme de solide brun fondant à 153°C.

Le chlorhydrate de 2-amino-6-chloro-1-indanone est préparé de la même façon que dans l'exemple 1 pour la préparation dde la 2-amino-1-indanone, à partir de 57,5 g de p-toluènesulfonate de 6-chloro-1-indanone-oxime, 3,94 g de sodium, 200 ml d'éthanol absolu et 1 litre de toluène anhydre. Après 15 heures de réaction à 0°C, le précipité formé est filtré sur célite et le filtrat lavé à l'eau, puis extrait avec de l'acide chlorhydrique 1N (2 x 500 ml) et la phase aqueuse concentrée à sec sous pression réduite. On obtient 10,5 g de produit attendu sous forme de solide brun-vert dont le point de fusion est supérieur à 260°C.

Le p-toluènesulfonate de 6-chloro-1-indanone-oxime peut être préparé suivant le protocole décrit dans l'exemple 1 pour la préparation du p-toluènesulfonate de 1-indanone-oxime, à partir de 33 g de 6-chloro-1-indanone-oxime, 69,3 g de chlorure de p-toluènesulfonyle et de 330 ml de pyridine. On obtient 57,5 g de produit attendu sous forme de solide beige fondant à 170°C.

La 6-chloro-indanone-oxime peut être préparée suivant le protocole décrit dans l'exemple 1 pour la préparation de la 1-indanone-oxime, à partir de 32,4 g de 6-chloro-1-indanone, 47,3 g de chlorhydrate d'hydroxylamine, 47 g de carbonate de potassium, 53 ml d'eau distillée et 530 ml de méthanol. On obtient 33 g de produit attendu sous forme ce solide jaune fondant à 167°C.

La 6-chloro-1-indanone peut être préparée selon le procédé décrit par R. Sieka et W. Kellermann, Chem. Ber., 75, 1730 (1942).

### EXEMPLE 3

On chauffe pendant 90 heures à 40°C un mélange de 0,7 g de (5-méthyl-2,3-dioxo-1,4-dihydro-5H-indéno[1,2-b]pyrazine-5-yl)acétate d'éthyle, 40 ml de dioxane et 9 ml d'acide chlorhydrique 8N. Le mélange réactionnel est ensuite évaporé au rotavapor et le résidu d'évaporation est trituré dans 20 ml d'eau, filtré et rincé à l'eau distillée (2x10 ml), puis à l'oxyde d'isopropyle (10 ml). Après séchage à 60°C sous vide (1 mmHg; 0,13 kPa) on obtient 0,4 g d'acide (5-méthyl-2,3-dioxo-1,4-dihydro-5H-indéno[1,2-b]pyrazine-5-yl)acétique sous forme de solide jaune pâle fondant au-dessus de 260°C (Analyse % calculé C : 61.,76, H : 4,44, N : 10,29, O : 23,51, % trouvé C : 61,8, H : 4,6, N : 10,4).

Le (5-méthyl-2,3-dioxo-1,4-dihydro-5H-indéno[1,2-b]pyrazine-5-yl)acétate d'éthyle peut être préparé de la façon suivante : on chauffe à reflux pendant 4 heures un mélange de 2,1 g de N-(1-éthoxycarbonylméthyl-1-méthyl-3-oxo-indane-2-yl)oxamate d'éthyle, 20 ml d'acide acétique et 4,6 g d'acétate d'ammonium. Le mélange réactionnel est ensuite concentré au rotavapor, additionné de 50 ml d'eau et soumis à deux extractions à l'acétate d'éthyle (2x50 ml). L'extrait organique est séché sur sulfate de magnésium, filtré et évaporé au rotavapor. L'huile jaune obtenue (2 g) est purifiée par cristallisation dans 15 ml d'acétonitrile. Après filtration et lavage des cristaux avec 2x10 ml d'acétonitrile et 2x10 ml d'oxyde d'isopropyle on obtient 0,7 g de (5-méthyl-2,3-dioxo-1,4-dihydro-5H-indéno[1,2-b]pyrazine-5-yl)acétate d'éthyle sous forme de solide jaune pâle (Spectre de R.M.N.¹H (300 MHz, (CD₃)₂SO d6, δ en ppm) : 0,77 (t, J = 7 Hz, 3H : CH₃ éthyle); 1,40 (s, 3H : CH₃); 3,00 (AB limite, 2H : CH₂); 3,20 (q, J = 7 Hz, 2H : COOCH₂ éthyle); 7,12 et 7,25 (2 t, J = 8 Hz, 2H : H 6 et H 7); 7,43 et 7,50 (2 d, J = 8 Hz, 2H : H 5 et H 8)).

Le N-(1-éthoxycarbonylméthyl-1-méthyl-3-oxo-indane-2-yl)oxamate d'éthyle peut être préparé de la manière suivante : on refroidit à une température voisine de -20°C un mélange de 5,5 g de (2-amino-1-méthyl-3-oxo-indan-1-yl)acétate d'éthyle chlorhydrate et 100 ml de dichlorométhane et on ajoute 2,6 ml de chlorure d'éthyloxalyle, puis lentement une solution de 6,1 ml de triéthylamine dans 20 ml de dichlorométhane en maintenant la température du milieu réactionnel proche de 0°C. A la fin de l'addition on laisse la température du mélange réactionnel remonter vers 20°C. Le mélange est alors filtré et le filtrat est lavé à l'eau distillée (2x80 ml). La solution organique est séchée sur sulfate de magnésium, filtrée et évaporée au rotavapor pour donner 6,2 g de N-(1-éthoxycarbonylméthyl)-1-méthyl-3-oxo-indane-2-yl)oxamate d'éthyle sous forme de d'huile brune (Spectre de R.M.N.¹H (200 MHz, (CD₃)₂SO d6, δ en ppm). On observe un mélange d'isomères 50 / 50 : 0,88-1,03 et 1,34 (3 t, J = 7 Hz, respectivement 1,5H - 1,5H et 3H : CH₃ éthyle); 1,21 et 1,65 (2 s, 1,5H chacun : CH₃); 2,75 et 3,03 (2 AB, respectivement J = 15 Hz et J = 16 Hz, 2H en totalité : CH₂); 3,78 - 3,92 et 4,32 (3 mts, 4H en totalité : COOCH₂ éthyle); 4,81 et 5,10 (2 d, J = 9 Hz, 1H en totolité : CH); de 7,40 à 7,85 (mt, 4H : H Aromatiques); 8,47 et 9,29 (2 d larges, J = 9 Hz, 1H en totalité : NHCO)).

Le (2-amino-1-méthyl-3-oxo-indan-1-yl)acétate d'éthyle chlorhydrate peut être préparé de la façon suivante : on sature d'acide chlorhydrique gazeux une solution de 7,32 g de (2-hydroxyimino-1-méthyl-3-oxo-indan-1-yl)acétate d'éthyle chlorhydrate dans 150 ml d'acide acétique et le mélange est ensuite hydrogéné pendant 20 heures sous une pression de 1,8 bar d'hydrogène à une température voisine de 20°C, en présence de 1,4 g de palladium sur charbon à 10%. Le mélange réactionnel est filtré et concentré au rotavapor. Le résidu d'évaporation est additionné de 50 ml d'acétate d'éthyle et à la solution brune obtenue on ajoute lentement 100 ml d'éther éthylique. Le précipité blanc qui apparaît est filtré, lavé à l'éther éthylique (2x50 ml) et séché. On obtient 5,5 g de (2-amino-1-méthyl-3-oxo-indan-1-yl)acétate d'éthyle chlorhydrate sous forme de solide blanc cassé fondant vers 172°C avec décomposition (Spectre de R.M.N.¹H (300 MHz, (CD₃)₂SO d6, δ en ppm). On observe un mélange d'isomères 60 / 40 : 0,86 - 1,06 (2 t, J = 7 Hz, 3H en totalité : CH₃ éthyle); 1,36 et 1,75 (2 s, 3H en totalité : CH₃); 2,92 - 3,20 et 3,46 (respectivement AB limite et 2 d (J = 16 Hz), 2H en totalité : CH₂); 3,80 et 3,96 (2 mts, 2H en totalité : COOCH₂ éthyle); 4,22 et 4,50 (2 s, 1H en totolité : CH); de 7,40 à 7,90 (mt, 4H : H Aromatiques); 8,94 (mf, 3H en totalité : NH₃⁺Cl⁻)).

Le (2-hydroxyimino-1-méthyl-3-oxo-indan-1-yl)acétate d'éthyle chlorhydrate peut être préparé selon le procédé décrit dans le brevet US 3703529.

### EXEMPLE 4

0,39 g de 5-acétamido-8-chloro-1,4-dihydro-5H-indéno[1,2-b]pyrazine-2,3-dione en suspension dans 5 ml d'acide chlorhydrique 6N sont portés à reflux pendant 5 heures. Après refroidissement à une température voisine de 20°C, l'insoluble est filtré, lavé à l'eau puis au méthanol, et repris dans 10 ml d'un mélange chaud d'eau et de méthanol (50/50 en volumes). Après quelques minutes d'agitation, l'insoluble est filtré et séché sous vide partiel (1 mm Hg; 0,13 kPa) pour conduire à 0,1 g de chlorhydrate de 5-amino-8-chloro-1,4-dihydro-5H-indéno[1,2-b]pyrazine-2,3-dione sous forme de solide gris dont le point de fusion est supérieur à 260°C (Spectre de R.M.N.¹H (250 MHz, (CD₃)₂SO d6, δ en ppm) : 5,15 (s, 1H : CH 9); 7,39 (dd, J = 8 et 2 Hz, 1H : H 7); 7,71 (d, J = 2 Hz, 1H : H 5); 7,75 (d, J = 8 Hz, 1H : H 8); de 8,70 à 9,20 (mf étalé , 3H : NH₃⁺Cl⁻); de 12,00 à 12,70 (mf étalé, 2H : NHCOCONH).

La 5-acétamido-8-chloro-1,4-dihydro-5H-indéno[1,2-b]pyrazine-2,3-dione peut être préparée selon la procédure suivante : à 1,74 g de 5-hydroxyimino-8-chloro-1,4-dihydro-indéno[1,2-b]pyrazine-2,3-dione en suspension dans 35 ml d'acide acétique, on ajoute par petites portions 1 g de zinc en poudre. Le milieu réactionnel est ensuite porté à 90°C pendant 1 heure. Après refroidissement à une température voisine de 20°C, on ajoute 0,7 ml d'anhydride acétique et on poursuit l'agitation à la même température pendant 48 heures. 50 ml d'eau distillée sont alors additionnés au milieu réactionnel et l'insoluble filtré, lavé à l'acétone et au méthanol pour conduire à 0,9 g de produit attendu sous forme de solide brun dont le point de fusion est supérieur à 260°C (Spectre de R.M.N.¹H (300 MHz, (CD₃)₂SO d6, δ en ppm) : 1,94 (s, 3H : COCH₃); 5,70 (d, J = 8 Hz, 1H : CH 9); 7,20 (dd, J = 8 et 2 Hz, 1H : H 7); 7,33 (d, J = 8 Hz, 1H : H 8); 7,60 (d, J = 2 Hz, 1H : H 5); 8,40 (d, J = 8 Hz, 1H : NHCO); 12,15 et 12,35 (2 mfs, 1H chacun : NHCOCONH).

La 5-hydroxyimino-8-chloro-1,4-dihydro-indéno[1,2-b]pyrazine-2,3-dione peut être préparée de la façon suivante : à 2,75 g de 8-chloro-1,4-dihydro-5H-indéno[1,2-b]pyrazine-2,3-dione en solution dans 45 ml de diméthylsulfoxyde, on ajoute par petites portions 1,07 g d'hydrure de sodium à 60%. Dès la fin du dégagement gazeux, 1,39 ml de nitrite d'isoamyle en solution dans 35 ml de diméthylsulfoxyde sont ajoutés au milieu réactionnel en environ 15 minutes. La réaction est poursuivie 1,5 heures à une température voisine de 20°C. Le milieu réactionnel est alors coulé sur 500 ml de glace, puis dilué avec 100 ml de méthanol. Le précipité est filtré et lavé au méthanol pour conduire à 2,44 g de produit attendu sous forme de solide noir utilisé sans purification supplémentaire dans les synthèses ultérieures.(Spectre de R.M.N.¹H (200 MHz, (CD₃)₂SO d6 avec ajout de quelques gouttes de CD₃COOD d4, δ en ppm). On observe le mélange des deux isomères Syn et Anti dans les proportions 65/35 : 7,23 (dd, J = 8 et 2 Hz, 1H : H 7); 7,51 et 8,01 (2 d, J = 8 Hz, 1H en totalité : H 8); 7,56 et 7,63 (2 d, J = 2 Hz, 1H en totalité : H 5).

### EXEMPLE 5

A une solution de 1,5 g de chlorhydrate de 7-amino-1,4-dihydro-5H-indéno[1,2-b]pyrazine-2,3-dione et de 2,48 ml de triéthylamine dans 20 ml de diméthylformamide, on ajoute 1,92 ml de phénylisocyanate à une température voisine de 20°C. Après une nuit d'agitation à la même température, le précipité formé est filtré, lavé à l'eau et séché sous vide partiel (1 mm Hg; 0,13 kPa) à 40°C pour conduire à 1,4 g de 7-(3-phényluréido)-1,4-dihydro-5H-indéno[1,2-b]pyrazine-2,3-dione sous forme de solide verdâtre dont le point de fusion est supérieur à 260°C (Analyse C18H14N4O3; 4,0 H2O % calculé C : 64,66; H : 4,22; N: 16,76; % trouvé C : 64,4; H : 4,5; N: 16,8; Spectre de R.M.N.¹H (250 MHz, (CD₃)₂SO d6, δ en ppm) : 3,58 (s, 2H : CH₂ 9); 7,00 (t, J = 7,5 Hz, 1H : H Aromatique du phényle en para de l'uréido); 7,30 (t, J = 7,5 Hz, 2H : H Aromatiques du phényle en méta de l'uréido); 7,50 (d, J = 7,5 Hz, 2H : H Aromatiques du phényle en ortho de l'uréido); de 7,20 à 7,60 (mt, 2H : H 5 et H 6); 7,72 (s large, 1H : H 8); 8,80 (s large, 2H : ArNHCONH); 12,00 et 12,28 (2 mfs, 1H chacun : NHCOCONH).

Le chlorhydrate de 7-amino-1,4-dihydro-5H-indéno[1,2-b]pyrazine-2,3-dione peut être obtenu de la manière suivante : à 6 g de 7-nitro-1,4-dihydro-5H-indéno[1,2-b]pyrazine-2,3-dione en suspension dans 135 ml de méthanol sont ajoutés goutte à goutte 100 ml d'acide chlorhydrique concentré, suivis par 4,27 g de fer en poudre et 27 ml de diméthylformamide. Le milieu réactionnel est agité 1 heure à température ambiante, puis à nouveau traité avec 4,27 g de fer en poudre et 27 ml de diméthylformamide. La réaction est poursuivie 2 heures à 80°C et après refroidissement à une température voisine de 20°C, l'insoluble est filtré, lavé au méthanol et séché pour conduire à 5,1 g de produit attendu sous forme de poudre ocre dont le point de fusion est supérieur à 260°C. (Spectre de R.M.N.¹H (250 MHz, (CD₃)₂SO d6 plus quelques gouttes de CD₃COOD d4, δ en ppm) : 3,60 (s, 2H : CH₂ 9); 7,34 (d large, J = 8 Hz, 1H : H 6); 7,47 (s large, 1H : H 8); 7,66 (d, J = 8 Hz, 1H : H 5).

La 7-nitro-1,4-dihydro-5H-indéno[1,2-b]pyrazine-2,3-dione peut être préparée selon la procédure suivante : à une solution de 10 g de 1,4-dihydro-5H-indéno[1,2-b]pyrazine-2,3-dione dans 170 ml d'acide sulfurique refroidie à -5°C, on ajoute 5,35 g de nitrate de potassium de façon à ce que la température n'excède pas 0°C. La réaction est poursuivie 3 heures à une température voisine de 20°C. Le milieu réactionnel est alors ajouté à de la glace et le précipité formé filtré, lavé à l'eau et séché pour conduire à 6 g de produit nitré attendu sous forme de poudre orangée dont le point de fusion est supérieur à 260°C (Spectre de R.M.N.¹H (300 MHz, (CD₃)₂SO d6, δ en ppm) : 3,73 (s, 2H : CH₂ 9); 7,71 (d, J = 8 Hz, 1H : H 5); 8,27 (dd, J = 8 et 2,5 Hz, 1H : H 6); 8,33 (s large, 1H : H 8); de 12,10 à 12,70 (mf, 2H : NHCOCONH).

### EXEMPLE 6

A 1 g de 8-chloro-1,4-dihydro-5H-indéno[1,2-b]pyrazine-2,3-dione et 0,59 g d'hydrate de l'acide glyoxylique en solution dans 15 ml de diméthylsulfoxyde, on ajoute progressivement 0,94 g d'hydrure de sodium en maintenant le milieu réactionnnel à une température voisine de 20°C. Après 18 heures de réaction à la même température, 5 ml d'acide acétique sont ajoutés au milieu réactionnel et le mélange porté à reflux pendant 4 heures. Après refroidissement, le mélange est filtré et l'insoluble noir lavé à l'eau, au méthanol puis repris dans 15 ml de méthanol et agité pendant une nuit à température ambiante. L'insoluble est filtré, lavé à l'acétone et séché sous vide partiel (1 mm Hg; 0,13 kPa) à 35°C. On obtient ainsi 0,7 g de (E)-5-carboxyméthylène-8-chloro-1,4-dihydro-indéno[1,2-b]pyrazine-2,3-dione sous forme de solide brun foncé dont le point de fusion est supérieur à 260°C. (Spectre de R.M.N.¹H (200 MHz, (CD₃)₂SO d6, δ en ppm) : 6,69 (s large, 1H : =CH); 7,15 (d large, J = 8,5 Hz, 1H : H 7); 7,54 (s large, 1H : H 5); 7,67 (d, J = 8,5 Hz, 1H : H 8); de 11,5 à 13,00 (mf étalé, 2H : OH); 15,90 (mf, 1H : COOH).

### EXEMPLE 7

Un mélange de 2,27 g de (8-chloro-5-méthyl-2,3-dioxo-1,4-dihydro-5H-indéno[1,2-b]pyrazine-5-yl)acétate d'éthyle, 120 ml de dioxane et de 25 ml d'une solution aqueuse d'acide chlorhydrique 8N est chauffé à une température voisine de 80°C pendant 4 heures. Le mélange réactionnel est ensuite évaporé sous pression réduite et le résidu d'évaporation est trituré dans 35 ml d'un mélange eau-éthanol (85/15 en volumes), filtré et lavé à l'eau distillée (2 fois 10 ml). Le produit obtenu est repris dans 68 ml d'une solution aqueuse 0,1N d'hydroxyde de sodium. L'insoluble est séparé par filtration et le filtrat est neutralisé par addition de 6,8 ml d'une solution aqueuse 1N d'acide chlorhydrique. Le solide obtenu est séparé par filtration, lavé à l'eau distillée (2 fois 5 ml) et séché à l'air. On obtient ainsi 0,41 g d'acide (8-chloro-5-méthyl-2,3-dioxo-1,4-dihydro-5H-indéno[1,2-b]pyrazine-5-yl)acétique sous forme d'un solide ocre fondant au dessus de 300°C [Spectre RMN ¹H (200 MHz, (CD₃)₂SO d6, δ en ppm ) : 1,39 (3H, s, CH₃); 2,93 (2H, m, -CH₂-CO); 7,19 (1H, dd, J = 8 et 2 Hz, H arom); 7,48 (1H, d, J = 8Hz, H arom); 7,56 (1H, d, 2=2Hz, H arom); 12,18 (1H, s, -NH-); 12,24, s, -NH); Analyse % calculé C : 54,83, H : 3,61, Cl : 11,56, N : 9,13, O : 20,87; % trouvé C : 55,2, H : 3,4, Cl : 11,2, N : 9,0, O : 18,6].

Le (8-chloro-5-méthyl-2,3-dioxo-1,4-dihydro-5H-indéno[1,2-b]pyrazine-5-yl)acétate d'éthyle peut être préparé de la même façon que celle décrite à l'exemple 3 pour la préparation du (5-méthyl-2,3-dioxo-1,4-dihydro-5H-indéno[1,2-b]pyrazine-5-yl)acétate d'éthyle mais à partir de 21,5 g de N-(5-chloro-1-éthoxycarbonylmethyl-1-méthyl-3-oxo-indane-2-yl)oxamate d'éthyle et de 43,6 g d'acétate d'ammonium dans 400 ml d'acide acétique. L'huile brute obtenue (32 g) est triturée dans 50 ml d'oxyde de diéthyle. On obtient ainsi après filtration du solide obtenu et lavage par 2 fois 10 ml d'oxyde de diéthyle 10 g de (8-chloro-5-méthyl-2,3-dioxo-1,4-dihydro-5H-indéno[1,2-b]pyrazine-5-yl)acétate d'éthyle soius forme d'un solide jaune utilisé tel quel dans les synthèses ultérieures.

Le N-(5-chloro-1-éthoxycarbonylméthyl-1-méthyl-3-oxo-indane-2-yl)oxamate d'éthyle peut être préparé de la même façon que celle décrite à l'exemple 3 pour la préparation du N-(1-éthoxycarbonylméthyl-1-méthyl-3-oxo-indane-2-yl)oxamate d'éthyle mais à partir de 18 g de (2-amino-5-chloro-1-méthyl-3-oxo-indan-1-yl)acétate d'éthyle chlorhydrate, de 7,6 ml de chlorure d'éthyloxalyle et de 17,5 ml de triéthylamine dans 200 ml de dichlorométhane. On obtient ainsi 20 g de N-(5-chloro-1-éthoxycarbonylméthyl-1-méthyl-3-oxo-indane-2-yl)oxamate d'éthyle sous forme d'une huile brune utilisée telle quelle dans les synthèses ultérieures.

Le (2-amino-5-chloro-1-méthyl-3-oxo-indan-1-yl)acétate d'éthyle chlorhydrate peut être préparé de la même façon que celle décrite à l'exemple 3 pour la préparation du (2-amino-1-méthyl-3-oxo-indan-1-yl)acétate d'éthyle chlorhydrate mais à partir de 20 g de (5-chloro-2-hydroxyimino-1-méthyl-3-oxo-indan-1-yl)acétate d'éthyle chlorhydrate dans 450 ml d'acide acétique saturée par de l'acide chlorhydrique gaz et en présence de 4 g de palladium sur charbon à 10%. On obtient ainsi après battage dans 200 ml d'oxyde de diéthyle 18,4 g de (2-amino-5-chloro-1-méthyl-3-oxo-indan-1-yl)acétate d'éthyle chlorhydrate sous forme d'un solide blanc-vert pâle fondant à 134°C.

Le (5-chloro-2-hydroxyimino-1-méthyl-3-oxo-indan-1-yl)acétate d'éthyle chlorhydrate peut être préparé de la façon suivante : à une solution de 40 g de (5-chloro-1-méthyl-3-oxo-indan-1-yl)acétate d'éthyle dans 150 ml d'éther éthylique et 55 ml d'une solution 5,5 N d'éther chlorhydrique refroidie à une température voisine de 10°C, on ajoute 26,7 ml de nitrite de tert-butyle. Le mélange réactionnel est agité 2 heures à une température voisine de 25°C puis concentré à sec sous pression réduite. Le résidu huileux obtenu est repris dans 30 ml d'acétate d'éthyle et évaporé à sec sous pression réduite. Cette dernière opération est réalisée 5 fois afin d'élimliner l'excès de nitrite de tert-butyle par distillation azéotropique. On obtient ainsi 43,7 g de (5-chloro-2-hydroxyimino-1-méthyl-3-oxo-indan-1-yl)acétate d'éthyle chlorhydrate sous forme d'un solide jaune pâle fondant à 144°C.

Le (5-chloro-1-méthyl-3-oxo-indan-1-yl)acétate d'éthyle peut être préparé de la façon suivante : à un mélange de 40 g d'acide (5-chloro-1-méthyl-3-oxo-indan-1-yl)acétique et de 0,5 ml de N,N-diméthylformamide dans 400 ml de dichlorométhane on ajoute lentement 15 ml de dichlorure d'oxalyle. Après 3 heures à une température voisine de 25°C on ajoute lentement 60 ml d'éthanol. Le mélange réactionnel est laissé 12 heures sous agitation puis est lavé par 2 fois 50 ml d'une solution aqueuse saturée d'hydrogénocarbonate de sodium, 50 ml d'eau distillée et 100 ml d'une solution aqueuse saturée de chlorure de sodium, séché sur sulfate de magnésium et concentré à sec sous pression réduite. On obtient ainsi 41 g de (5-chloro-1-méthyl-3-oxo-indan-1-yl)acétate d'éthyle sous forme d'une huile brune utilisée telle quelle dans les synthèses ultérieures.

L'acide (5-chloro-1-méthyl-3-oxo-indan-1-yl)acétique peut être préparé de la façon suivante : un mélange de 74 g d'acide 3-(4-chlorophényl)-3-méthylglutarique dans 300 ml d'acide sulfurique concentré est chauffé au reflux pendant 24 heures. Après retour à une température voisine de 25°C, le milieu réactionnel est versé lentement sur 1 litre d'eau glacée puis est extrait par 3 fois 500 ml d'acétate d'éthyle. Les phases organiques réunies sont lavées par 3 fois 500 ml d'eau, 500ml d'une solution aqueuse saturée de chlorure de sodium, séchées sur sulfate de magnésium et concentrées à sec sous pression réduite. L'huile brute (60 g) est triturée dans 50 ml d'éther éthylique. Le solide obtenu est séparé par filtration, lavé par 20 ml d'éther éthylique et séché à l'air. On obtient ainsi 48 g d'acide (5-chloro-1-méthyl-3-oxo-indan-1-yl)acétique sous forme d'un solide crème fondant à 119°C.

L'acide 3-(4-chlorophényl)-3-méthyl-glutarique peut être préparé de la façon suivante : une solution de 110 g de 2,4-dicyano-3-méthyl-3-(4-chlorophényl)glutarimide dans un mélange constitué de 470 ml d'eau, 470 ml d'acide sulfurique concentré et de 300 ml d'acide acétique est chauffée au reflux pendant 42 heures. Après retour à une température voisine de 25°C, le milieu réactionnel est versé sur 2 litres d'eau glacée. Le précipité formé est séparé par filtration, lavé par 2 fois 50 ml d'eau distillée et séché à l'air. On obtient ainsi 76,3 g d'acide 3-(4-chlorophényl)-3-méthylpentanedioïque sous forme d'un solide crème fondant à 128°C.

Le 2,4-dicyano-3-méthyl-3-(4-chlorophényl)glutarimide peut être préparé de la façon suivante : à une solution d'éthylate de sodium éthanolique obtenue par addition de 10,2 g de sodium dans 400 ml d'éthanol on ajoute lentement, à une température voisine de 5°C, 37 g de cyanoacétamide. Après 15 minutes on ajoute à la suspension obtenue une solution de 110 g de 2-cyano-3-(4-chlorophényl)-2-butène-oate d'éthyle dans 300 ml d'éthanol. Le mélange réactionnel est agité pendant 4 heures à une température voisine de 25°C puis est versé sur 500 ml d'eau distillée. Le milieu réactionnel est refroidi à une température voisine de 5°C puis acidifié par addition de 85 ml d'une solution aqueuse d'acide chlorhydrique concentré. Le précipité est séparé par filtration, lavé par 2 fois 50 ml d'eau et séché à l'air. On obtient ainsi 112 g de 2,4-dicyano-3-méthyl-3-(4-chlorophényl)-glutarimide sous forme d'un solide jaune fondant à 258°C.

Le 2-cyano-3-(4-chlorophényl)-2-butène-oate d'éthyle peut être préparé de la façon suivante : un mélange de 130 ml de 4-chloroacétophénone, de 107 ml de cyanoacétate d'éthyle, de 15,4 g d'acétate d'ammonium et de 48 ml d'acide acétique dans 200 ml de toluène est chauffé au reflux pendant 12 heures en éliminant l'eau formée au cours de la réaction par distillation azéotropique. Après retour à une temperature voisine de 25°C, le mélange réactionnel est dilué par 100 ml de toluène, lavé par 2 fois 100 ml d'eau distillée et par 100 ml d'une solution aqueuse saturée de chlorure de sodium, séché sur sulfate de magnésium et concentré à sec sous pression réduite. L'huile brute obtenue est distillée sous pression réduite (pression voisine de 30 mm de Hg) et on collecte la fraction distillant entre 140 et 180°C. On obtient ainsi 110 g de 2-cyano-3-(4-chlorophényl)-2-butène-oate d'éthyle sous forme d'une huile jaune épaisse utilisée telle quelle dans les synthèses ultérieures.

### EXEMPLE 8

On procède comme à l'exemple 3, mais à partir de 1,2 g de (+) (5-méthyl-2,3-dioxo-1,4-dihydro-1H-indéno[1,2-b]pyrazine-5-yl)acétate d'éthyle, 70 ml de dioxane et 15 ml d'acide chlorhydrique 8N. L'acide obtenu (0,64 g) est purifié par solubilisation dans 40 ml d'eau distillée et 0,2 g d'hydrogénocarbonate de sodium, lavage de cette solution avec 15 ml d'acétate d'éthyle et acidification de la phase aqueuse avec 2,5 ml d'acide chlorhydrique 1N. Après addition de 12 g de chlorure de sodium, le précipité formé est filtré, lavé avec 5 ml d'eau distillée, puis 10 ml d'éther isopropylique et séché à 60°C sous vide (1 mmHg; 0,13 kPa). L'acide ainsi purifié (0,27 g) est transformé en sel de sodium par mise en solution dans 10 ml de soude 0,1N, filtration et lyophilisation. On obtient 0,28 g de (+) acide (5-méthyl-2,3-dioxo-1,4-dihydro-1H-indéno[1,2-b]pyrazine-5-yl)acétique sous forme de sel de sodium fondant au-dessus de 260°C (Analyse % calculé C : 57,15, H : 3,77, N : 9,52, Na : 7,81, O : 21,75, % trouvé C : 57,1, N : 9,0; α_{D}²⁰ = + 24,1 (eau; c = 0,5%)).

En procédant comme précédemment mais à partir de 1,2 g de (-) (5-méthyl-2,3-dioxo-1,4-dihydro-1H-indéno[1,2-b]pyrazine-5-yl)acétate d'éthyle, 70 ml de dioxane et 15 ml d'acide chlrohydrique 8N, on obtient 0,27 g de (-) acide (5-méthyl-2,3-dioxo-1,4-dihydro-1H-indéno[1,2-b]pyrazine-5-yl)acétique sous forme de sel de sodium fondant au-dessus de 260°C (Analyse % calculé C : 57,15, H : 3,77, N : 9,52, Na : 7,81, O : 21,75, % trouvé C : 57,2, H : 3,8, N : 9,1; α_{D}²⁰ = - 23,6 (eau; c = 0,5%)).

Le (+) (5-méthyl-2,3-dioxo-1,4-dihydro-1H-indéno[1,2-b]pyrazine-5-yl)acétate d'éthyle et le (-) (5-méthyl-2,3-dioxo-1,4-dihydro-1H-indéno[1,2-b]pyrazine-5-yl)acétate d'éthyle sont préparés par chromatographie du produit racémique sur colonne chirale CHIRACEL OD DAICEL (250 mm de longueur et 60 mm de diamètre) en utilisant comme éluant un mélange éthanol-heptane (70-30 en volumes) avec un débit de 80 ml/minute. A partir de 3x1,1 g de (+/-) (5-méthyl-2,3-dioxo-1,4-dihydro-1H-indéno[1,2-b]pyrazine-5-yl)acétate d'éthyle on obtient 1,57 g de (+) (5-méthyl-2,3-dioxo-1,4-dihydro-1H-indéno[1,2-b]pyrazine-5-yl)acétate d'éthyle sous forme de solide jaune (α_{D}²⁰ = + 51,2 (méthanol; c = 0,5%)) et 1,52 g de (-) (5-méthyl-2,3-dioxo-1,4-dihydro-1H-indéno[1,2-b]pyrazine-5-yl)acétate d'éthyle sous forme de solide jaune (α_{D}²⁰ = + 24,1 (eau; c = 0,5%)).

### EXEMPLE 9

En procédant comme à l'exemple 8 mais à partir du (+) (8-chloro-5-méthyl-2,3-dioxo-1,4-dihydro-5H-indéno[1,2-b]pyrazine-5-yl)acétate d'éthyle on obtient le (+) acide (8-chloro-5-méthyl-2,3-dioxo-1,4-dihydro-5H-indéno[1,2-b]pyrazine-5-yl)acétique et en partant du (-) (8-chloro-5-méthyl-2,3-dioxo-1,4-dihydro-5H-indéno[1,2-b]pyrazine-5-yl)acétate d'éthyle on obtient le (-) acide (8-chloro-5-méthyl-2,3-dioxo-1,4-dihydro-5H-indéno[1,2-b]pyrazine-5-yl)acétique.

Le (+) (8-chloro-5-méthyl-2,3-dioxo-1,4-dihydro-5H-indéno[1,2-b]pyrazine-5-yl)acétate d'éthyle et le (-) (8-chloro-5-méthyl-2,3-dioxo-1,4-dihydro-5H-indéno[1,2-b]pyrazine-5-yl)acétate d'éthyle sont préparés comme décrit à l'exemple 8 à partir du mélange (+/-) (8-chloro-5-méthyl-2,3-dioxo-1,4-dihydro-5H-indéno[1,2-b]pyrazine-5-yl)acétate d'éthyle.

Les médicaments selon l'invention sont constitués par un composé de formule (I) ou un sel d'un tel composé, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

En thérapeutique humaine, les composes selon l'invention sont particulièrement utiles pour le traitement et/ou la prévention des conditions qui requièrent l'administration d'un antagoniste du récepteur AMPA ou d'un antagoniste du récepteur NMDA. Ces composés sont notamment utiles pour traiter ou prévenir toutes les ischémies et en particulier l'ischémie cérébrale, les effets dus à une anoxie, l'évolution de maladies neurodégénératives, de la chorée d'HUNTINGTON, de la maladie d'ALZHEIMER, de la sclérose latérale amyotrophique, de l'atrophie olivo-pontocérébelleuse et de la maladie de PARKINSON, vis-à-vis des manifestations épileptogènes et/ou convulsives, pour le traitement des traumatismes cérébraux et spinaux, des traumatismes liés à le dégénérescence de l'oreille interne ou de la rétine, de l'anxiété, de la dépression, de la schizophrénie, du syndrome de TOURETTE, de l'encéphalopathie hépatique, en tant qu'analgésiques, antiinflammatoires, antianorexiques, antimigraineux, antiémétiques et pour traiter les empoisonnements par des neurotoxines ou d'autres substances agonistes du récepteur NMDA, ainsi que les troubles neurologiques associés aux maladies virales telles que le sida, la rage, la rougeole et le tétanos. Ces composés sont aussi utiles pour la prévention des symptomes d'abstinence aux drogues et à l'alcool et de l'inhibition de l'accoutumance et de la dépendance aux opiacés ainsi que pour le traitement des déficits liés à des anomalies mitochondriales tells que la myopathie mitochondriale, le syndrome de Leber, l'encéphalopathies de Wernicke, le syndrome de Rett, l'homocystéinémie, l'hyperprolinémie, l'hydroxybutirique-aminoacidurie, l'encéphalopathie saturnine (intoxication chronique au plomb) et la déficience en sulfite oxydase.

Les doses dépendent de l'effet recherche, de la durée du traitement et de la voie d'administration utilisée; elles sont généralement comprises entre 10 mg et 100 mg par jour par voie orale pour un adulte avec des doses unitaires allant de 5 mg à 50 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :

| | |
|---|---|
| - Composé de formule (I) | 50 mg |
| - Cellulose | 18 mg |
| - Lactose | 55 mg |
| - Silice colloïdale | 1 mg |
| - Carboxyméthylamidon sodique | 10 mg |
| - Talc | 10 mg |
| - Stéarate de magnésium | 1 mg |

### EXEMPLE B

On prépare selon la technique habituelle des comprimés dosés à 50 mg de produit actif ayant la composition suivante :

| | |
|---|---|
| - Composé de formule (I) | 50 mg |
| - Lactose | 104 mg |
| - Cellulose | 40 mg |
| - Polyvidone | 10 mg |
| - Carboxyméthylamidon sodique | 22 mg |
| - Talc | 10 mg |
| - Stéarate de magnésium | 2 mg |
| - Silice colloïdale | 2 mg |
| - Mélange d'hydroxyméthylcellulose, glycérine, oxyde de titane (72-3,5-24,5) q.s.p. 1 comprimé pelliculé terminé à | 245 mg |

### EXEMPLE C

On prépare une solution injectable contenant 10 mg de produit actif ayant la composition suivante :

| | |
|---|---|
| - Composé de formule (I) | 10 mg |
| - Acide benzoïque | 80 mg |
| - Alcool benzylique | 0,06 ml |
| - Benzoate de sodium | 80 mg |
| - Ethanol à 95 % | 0,4 ml |
| - Hydroxyde de sodium | 24 mg |
| - Propylène glycol | 1,6 ml |
| - Eau q.s.p. | 4 ml |

## Revendications

1. Composés de formule : 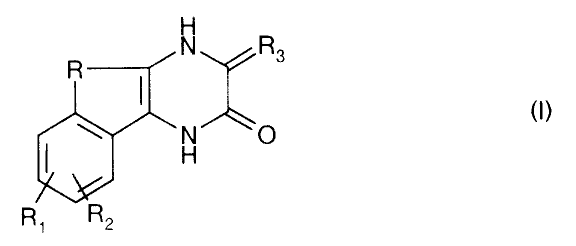 dans laquelle
- R représente un radical C(R₄)R₅, CH-R₆ ou C=R₇,
- R₁ et R₂, identiques ou différents, représentent des atomes d'hydrogène ou d'halogène ou des radicaux amino, nitro ou -NH-CO-NR₁₁R₁₂,
- R₃ représente un atome d'oxygène,
- R₄ représente un radical alkyle,
- R₅ représente un radical -alk-COOR₁₀,
- R₆ représente un atome d'hydrogène ou un radical -NR₁₄R₁₅,
- R₇ représente un radical NOH ou C(COOR₁₀)R₂₀,
- R₁₀ représente un atome d'hydrogène ou un radical alkyle,
- R₁₁ représente phényle,
- R₁₂ représente un atome d'hydrogène,
- R₁₄ représente un atome d'hydrogène,
- R₁₅ représente un atome d'hydrogène ou un radical -COR₂₂,
- R₂₀ représente un atome d'hydrogène,
- R₂₂ représente un radical alkyle,
- alk représente un radical alkyle,
étant entendu que lorsque R₁ et R₂ représentent des atomes d'hydrogène et R₃ représente un atome d'oxygène, R n'est pas un radical C=R₇ dans lequel R₇ représente un radical NOH,
étant entendu que les radicaux et portions alkyle, contiennent 1 à 6 atomes de carbone et sont en chaîne droite ou ramifiée,
ainsi que les isomères E et Z des composés pour lesquels R représente un radical C=R₇ pour lequel R₇ représente un radical C(COOR₁₀)R₂₀, les énantiomères et diastéréoisomères des composés pour lesquels R représente un radical C(R₄)R₅ ou CH-R₆,
ainsi que leurs sels.

2. Composés de formule (I) selon la revendication 1 suivants :
- 1,4-dihydro-5H-indéno[1,2-b]pyrazine-2,3-dione,
- 8-chloro-1,4-dihydro-5H-indeno[1,2-b]pyrazine-2,3-dione,
- acide (5-méthyl-2,3-dioxo-1,4-dihydro-5H-indéno[1,2-b]pyrazine-5-yl)acétique,
- 5-amino-8-chloro-1,4-dihydro-5H-indéno[1,2-b]pyrazine-2,3-dione,
- 7-(3-phényluréido)-1,4-dihydro-5H-indeno[1,2-b]pyrazine-2,3-dione,
- (E)-5-carboxyméthylène-8-chloro-1,4-dihydro-indéno[1,2-b]pyrazine-2,3-dione,
- acide (8-chloro-5-méthyl-2,3-dioxo-1,4-dihydro-5H-indéno[1,2-b]pyrazine-5-yl)acétique,
- (+) acide (5-méthyl-2,3-dioxo-1,4-dihydro-1H-indéno[1,2-b]pyrazine-5-yl)acétique,
- (-) acide (5-méthyl-2,3-dioxo-1,4-dihydro-1H-indéno[1,2-b]pyrazine-5-yl)acétique,
- (+) acide (8-chloro-5-méthyl-2,3-dioxo-1,4-dihydro-5H-indéno[1,2-b]pyrazine-5-yl)acétique.
- (-) acide (8-chloro-5-méthyl-2,3-dioxo-1,4-dihydro-5H-indéno[1,2-b]pyrazine-5-yl)acétique
et leurs sels.

3. Procédé de préparation des composés de formule (I) selon la revendication 1 pour lesquels R représente un radical C(R₄)R₅ ou CH-R₆, R₆ représente un atome d'hydrogène et R₃ représente un atome d'oxygène caractérisé en ce que l'on cyclise, en présence d'acétate d'ammonium, un dérivé de formule : 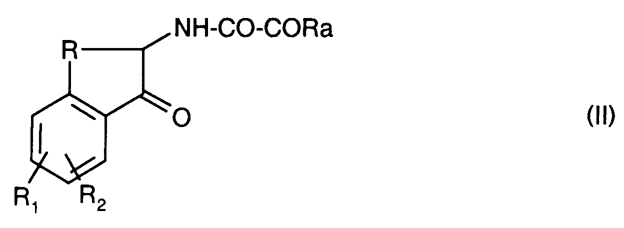 dans laquelle R représente un radical C(R₄)R₅ ou CH-R₆ dans lequel R₆ représente un atome d'hydrogène, R₁ et R₂ ont les mêmes significations que dans la revendication 1 et Ra représente un radical alcoxy, isole le produit et le transforme éventuellement en sel.

4. Procédé de préparation des composés de formule (I) selon la revendication 1 pour lesquels R représente un radical C=R₇ et R₇ représente un radical NOH caractérisé en ce que l'on fait réagir un nitrite d'alkyle sur un composé de formule (I) correspondant pour lequel R représente un radical CH-R₆ et R₆ représente un atome d'hydrogène, isole le produit et le transforme éventuellement en sel.

5. Procédé de préparation des composés de formule (I) selon la revendication 1 pour lesquels R représente un radical C=R₇ et R₇ représente un radical C(COOR₁₀)R₂₀ caractérisé en ce que l'on déshydrate un dérivé de formule : 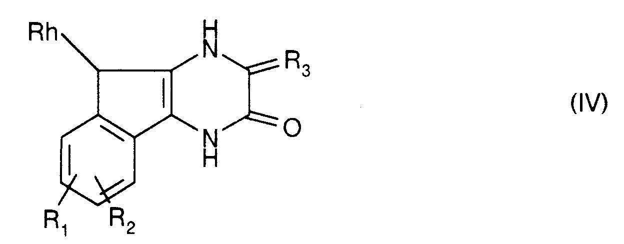 dans laquelle R₁, R₂ et R₃ ont les mêmes significations que dans la revendication 1 et Rh représente un radical -C(R₂₀)(OH)-COOR₁₀ dans lequel R₂₀ et R₁₀ ont les mêmes significations que dans la revendication 1, isole le produit et le transforme éventuellement en sel.

6. Procédé de préparation des composés de formule (I) selon la revendication 1 pour lesquels R représente un radical CH-R₆ dans lequel R₆ représente un radical -NR₁₄R₁₅, R₁₄ et R₁₅ représentent chacun un atome d'hydrogène caractérisé en ce que l'on hydrolyse un composé de formule (I) correspondant pour lequel R représente un radical CH-R₆ dans lequel R₆ représente un radical -NR₁₄R₁₅, R₁₄ représente un atome d'hydrogène et R₁₅ représente un radical -COR₂₂ et R₂₂ représente un radical alkyle, isole le produit et le transforme éventuellement en sel.

7. Procédé de préparation des composés de formule (I) selon la revendication 1 pour lesquels R représente un radical CH-R₆ dans lequel R₆ représente un radical -NR₁₄R₁₅, R₁₄ représente un atome d'hydrogène, R₁₅ représente un radical -COR₂₂ et R₂₂ représente un radical alkyle caractérisé en ce que l'on fait réagir un agent réducteur sur un composé de formule (I) correspondant pour lequel R représente un radical C=R₇ et R₇ représente un radical NOH, puis d'un traitement avec un anhydride (RqCO)₂O pour lequel Rq représente un radical alkyle, isole le produit et le transforme éventuellement en sel.

8. Procédé de préparation des composes de formule (I) selon la revendication 1 pour lesquels R₁ et éventuellement R₂ représentent un radical -NH-CO-NR₁₁R₁₂ et R₁₂ représente un atome d'hydrogène caractérisé en ce que l'on fait réagir un composé de formule (I) correspondant pour lequel R₁ représente un radical amino sur un dérivé Rx=C=N-Ry dans lequel Rx représente un atome d'oxygène et Ry représente un radical phényle, isole le produit et le transforme éventuellement en sel.

9. Médicaments contenant en tant que principe actif au moins un composé de formule (I) selon l'une des revendications 1 ou 2 ou un sel pharmaceutiquement acceptable d'un tel composé.

## Claims

1. Compounds of formula: 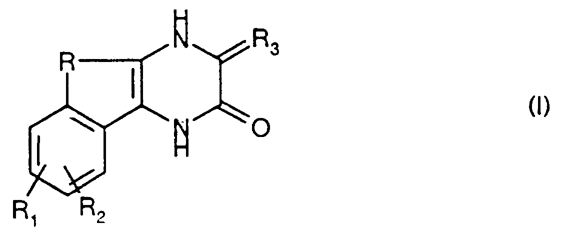 in which
- R represents a C(R₄)R₅, CH-R₆ or C=R₇ radical,
- R₁ and R₂, which may be identical or different, represent hydrogen or halogen atoms or amino, nitro or -NH-CO-NR₁₁R₁₂ radicals,
- R₃ represents an oxygen atom,
- R₄ represents an alkyl radical,
- R₅ represents an -alk-COOR₁₀ radical,
- R₆ represents a hydrogen atom or an -NR₁₄R₁₅ radical,
- R₇ represents an NOH or C(COOR₁₀)R₂₀ radical,
- R₁₀ represents a hydrogen atom or an alkyl radical,
- R₁₁ represents phenyl,
- R₁₂ represents a hydrogen atom,
- R₁₄ represents a hydrogen atom,
- R₁₅ represents a hydrogen atom or a -COR₂₂ radical,
- R₂₀ represents a hydrogen atom,
- R₂₂ represents an alkyl radical,
- alk represents an alkyl radical,
on the understanding that, when R₁ and R₂ represent hydrogen atoms and R₃ represents an oxygen atom, R is not a radical C=R₇ in which R₇ represents an NOH radical,
on the understanding that the alkyl radicals and portions contain 1 to 6 carbon atoms and are unbranched- or branched-chain radicals,
as well as the E and Z isomers of the compounds for which R represents a C=R₇ radical for which R₇ represents a C(COOR₁₀)R₂₀ radical, the enantiomers and diastereoisomers of the compounds for which R represents a C(R₄)R₅ or CH-R₆ radical,
as well as their salts.

2. Compounds of formula (I) according to claim 1 as follows:
- 1,4-dihydro-5H-indeno[1,2-b]pyrazine-2,3-dione,
- 8-chloro-1,4-dihydro-5H-indeno[1,2-b]pyrazine-2,3-dione,
- (5-methyl-2,3-dioxo-1,4-dihydro-5H-indeno[1,2-b]pyrazin-5-yl)acetic acid,
- 5-amino-8-chloro-1,4-dihydro-5H-indeno[1,2-b]pyrazine-2,3-dione,
- 7-(3-phenylureido)-1,4-dihydro-5H-indeno[1,2-b]pyrazine-2,3-dione,
- (E)-5-carboxymethylene-8-chloro-1,4-dihydroindeno[1,2-b]pyrazine-2,3-dione,
- (8-chloro-5-methyl-2,3-dioxo-1,4-dihydro-5H-indeno[1,2-b]pyrazin-5-yl)acetic acid,
- (+)-(5-methyl-2,3-dioxo-1,4-dihydro-1H-indeno[1,2-b]pyrazin-5-yl)acetic acid,
- (-)-(5-methyl-2,3-dioxo-1,4-dihydro-1H-indeno[1,2-b]pyrazin-5-yl)acetic acid,
- (+)-(8-chloro-5-methyl-2,3-dioxo-1,4-dihydro-5H-indeno[1,2-b]pyrazin-5-yl)acetic acid,
- (-)-(8-chloro-5-methyl-2,3-dioxo-1,4-dihydro-5H-indeno[1,2-b]pyrazin-5-yl)acetic acid,
and their salts.

3. Process for preparing the compounds of formula (I) according to claim 1 for which R represents a C(R₄)R₅ or CH-R₆ radical, R₆ represents a hydrogen atom and R₃ represents an oxygen atom, characterized in that a derivative of formula: 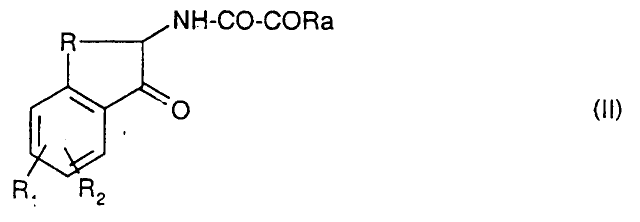 in which R represents a C(R₄)R₅ or CH-R₆ radical in which R₆ represents a hydrogen atom, R₁ and R₂ have the same meanings as in claim 1 and Ra represents an alkoxy radical, is cyclized in the presence of ammonium acetate, and the product is isolated and optionally converted to a salt.

4. Process for preparing the compounds of formula (I) according to claim 1 for which R represents a C=R₇ radical and R₇ represents an NOH radical, characterized in that an alkyl nitrite is reacted with a corresponding compound of formula (I) for which R represents a CH-R₆ radical and R₆ represents a hydrogen atom, and the product is isolated and optionally converted to a salt.

5. Process for preparing the compounds of formula (I) according to claim 1 for which R represents a C=R₇ radical and R₇ represents a C(COOR₁₀)R₂₀ radical, characterized in that a derivative of formula: 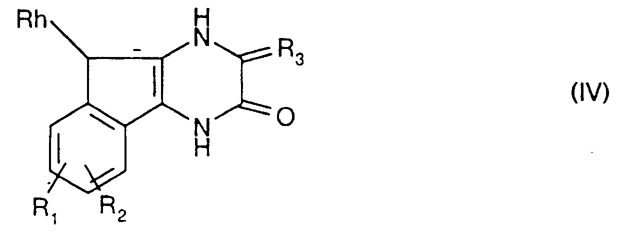 in which R₁, R₂ and R₃ have the same meanings as in claim 1 and Rh represents a -C(R₂₀)(OH)-COOR₁₀ radical in which R₂₀ and R₁₀ have the same meanings as in claim 1, is dehydrated, and the product is isolated and optionally converted to a salt.

6. Process for preparing the compounds of formula (I) according to claim 1 for which R represents a CH-R₆ radical in which R₆ represents an -NR₁₄R₁₅ radical, R₁₄ and R₁₅ each represent a hydrogen atom, characterized in that a corresponding compound of formula (I) for which R represents a CH-R₆ radical, in which R₆ represents an -NR₁₄R₁₅ radical, R₁₄ represents a hydrogen atom and R₁₅ represents a -COR₂₂ radical and R₂₂ represents an alkyl radical, is hydrolysed, and the product is isolated and optionally converted to a salt.

7. Process for preparing the compounds of formula (I) according to claim 1 for which R represents a radical CH-R₆ in which R₆ represents a radical -NR₁₄R₁₅, R₁₄ represents a hydrogen atom, R₁₅ represents a radical -COR₂₂ and R₂₂ represents an alkyl radical, characterized in that a reducing agent is reacted with a corresponding compound of formula (I) for which R represents a radical C=R₇ and R₇ represents an NOH radical, followed by treatment with an anhydride (RqCO)₂O for which Rq represents an alkyl radical, and the product is isolated and optionally converted to a salt.

8. Process for preparing the compounds of formula (I) according to claim 1 for which R₁ and optionally R₂ represent a radical -NH-CO-NR₁₁R₁₂ and R₁₂ represents a hydrogen atom, characterized in that a corresponding compound of formula (I) for which R₁ represents an amino radical is reacted with a derivative Rx=C=N-Ry in which Rx represents an oxygen atom and Ry represents a phenyl radical, and the product is isolated and optionally converted to a salt.

9. Medicinal products containing as active principle at least one compound of formula (I) according to either of claims 1 and 2 or a pharmaceutically acceptable salt of such a compound.

## Patentansprüche

1. Verbindungen der Formel: 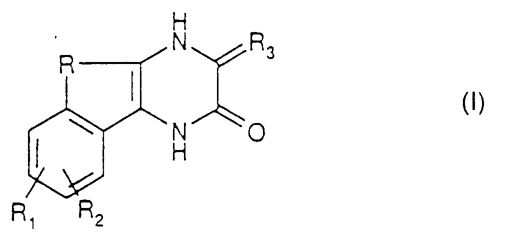
- in der R einen C(R₄)R₅-, CH-R₆- oder C=R₇-Rest darstellt,
- R₁ und R₂, die gleich oder verschieden sind, Wasserstoff- oder Halogenatome oder Amino-, Nitro- oder -NH-CO-NR₁₁R₁₂-Reste darstellen,
- R₃ ein Sauerstoffatom darstellt,
- R₄ einen Alkylrest darstellt,
- R₅ einen -alk-COOR₁₀-Rest darstellt,
- R₆ ein Wasserstoffatom oder einen -NR₁₄R₁₅-Rest darstellt,
- R₇ einen NOH- oder C(COOR₁₀)R₂₀-Rest darstellt,
- R₁₀ ein Wasserstoffatom oder einen Alkylrest darstellt,
- R₁₁ Phenyl darstellt,
- R₁₂ ein Wasserstoffatom darstellt,
- R₁₄ ein Wasserstoffatom darstellt,
- R₁₅ ein Wasserstoffatom oder einen -COR₂₂-Rest darstellt,
- R₂₀ ein Wasserstoffatom darstellt,
- R₂₂ einen Alkylrest darstellt,
- alk einen Alkylrest darstellt,
wobei es sich versteht, daß, wenn R₁ und R₂ Wasserstoffatome darstellen und R₃ ein Sauerstoffatom darstellt, R kein C=R₇-Rest ist, in dem R₇ einen NOH-Rest darstellt,
wobei es sich versteht, daß die Alkylreste und -teile 1 bis 6 Kohlenstoffatome enthalten und gerad- oder verzweigtkettig sind,
sowie die E- und Z-Isomere der Verbindungen, für die R einen C=R₇-Rest darstellt, für den R₇ einen C(COOR₁₀)R₂₀-Rest darstellt, die Enantiomere und Diastereoisomere der Verbindungen, für die R einen C(R₄)R₅- oder CH-R₆-Rest darstellt,
sowie deren Salze.

2. Folgende Verbindungen der Formel (I) gemäß Anspruch 1:
- 1,4-Dihydro-5H-indeno[1,2-b]pyrazin-2,3-dion,
- 8-Chloro-1,4-dihydro-5H-indeno[1,2-b]pyrazin-2,3-dion,
- (5-Methyl-2,3-dioxo-1,4-dihydro-5H-indeno[1,2-b]pyrazin-5-yl)essigsäure,
- 5-Amino-8-chloro-1,4-dihydro-5H-indeno[1,2-b]pyrazin-2,3-dion,
- 7-(3-Phenylureido)-1,4-dihydro-5H-indeno[1,2-b]pyrazin-2,3-dion,
- (E)-5-Carboxymethylen-8-chloro-1,4-dihydro-indeno[1,2-b]pyrazin-2,3-dion,
- (8-Chloro-5-methyl-2,3-dioxo-1,4-dihydro-5H-indeno[1,2-b]pyrazin-5-yl)essig säure,
- (+)-(5-Methyl-2,3-dioxo-1,4-dihydro-1H-indeno[1,2-b]pyrazin-5-yl)essigsäure,
- (-)-(5-Methyl-2,3-dioxo-1,4-dihydro-1H-indeno[1,2-b]pyrazin-5-yl)essigsäure,
- (+)-(8-Chloro-5-methyl-2,3-dioxo-1,4-dihydro-5H-indeno[1,2-b]pyrazin-5-yl)essig säure,
- (-)-(8-Chloro-5-methyl-2,3-dioxo-1,4-dihydro-5H-indeno[1,2-b]pyrazin-5-yl)essig säure,
und deren Salze.

3. Verfahren zur Herstellung der Verbindungen der Formel (I) gemäß Anspruch 1, für die R einen C(R₄)R₅- oder CH-R₆-Rest darstellt, R₆ ein Wasserstoffatom darstellt und R₃ ein Sauerstoffatom darstellt, dadurch gekennzeichnet, daß man ein Derivat der Formel: 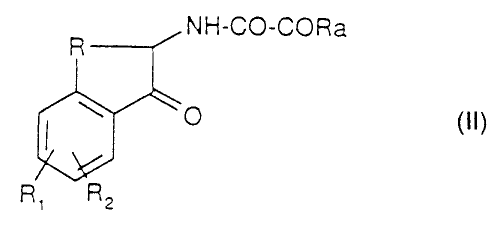 in der R einen C(R₄)R₅- oder CH-R₆-Rest, in dem R₆ ein Wasserstoffatom darstellt, darstellt, R₁ und R₂ die gleichen Bedeutungen wie in Anspruch 1 haben und Ra einen Alkoxyrest darstellt, in Gegenwart von Ammoniumacetat cyclisiert, das Produkt isoliert und es gegebenenfalls in ein Salz überführt.

4. Verfahren zur Herstellung der Verbindungen der Formel (I) gemäß Anspruch 1, für die R einen C=R₇-Rest darstellt und R₇ einen NOH-Rest darstellt, dadurch gekennzeichnet, daß man ein Alkylnitrit mit einer entsprechenden Verbindung der Formel (I), für die R einen CH-R₆-Rest darstellt und R₆ ein Wasserstoffatom darstellt, umsetzt, das Produkt isoliert und es gegebenenfalls in ein Salz überführt.

5. Verfahren zur Herstellung der Verbindungen der Formel (I) gemäß Anspruch 1, für die R einen C=R₇-Rest darstellt und R₇ einen C(COOR₁₀)R₂₀-Rest darstellt, dadurch gekennzeichnet, daß man ein Derivat der Formel: 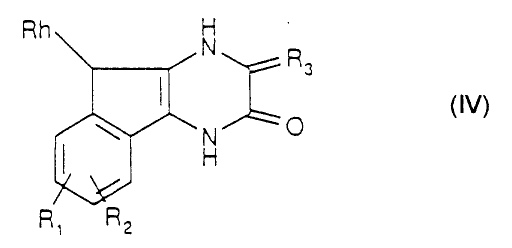 in der R₁, R₂ und R₃ die gleichen Bedeutungen wie in Anspruch 1 haben und Rh einen -C(R₂₀)(OH)-COOR₁₀-Rest darstellt, in dem R₂₀ und R₁₀ die gleichen Bedeutungen wie in Anspruch 1 haben, dehydratisiert, das Produkt isoliert und es gegebenenfalls in ein Salz überführt.

6. Verfahren zur Herstellung der Verbindungen der Formel (I) gemäß Anspruch 1, für die R einen CH-R₆-Rest darstellt, in dem R₆ einen -NR₁₄R₁₅-Rest darstellt, R₁₄ und R₁₅ jedes ein Wasserstoffatom darstellen, dadurch gekennzeichnet, daß man eine entsprechende Verbindung der Formel (I), für die R einen CH-R₆-Rest darstellt, in dem R₆ einen -NR₁₄R₁₅-Rest darstellt, R₁₄ ein Wasserstoffatom darstellt und R₁₅ einen -COR₂₂-Rest darstellt und R₂₂ einen Alkylrest darstellt, hydrolysiert, das Produkt isoliert und es gegebenenfalls in ein Salz überführt.

7. Verfahren zur Herstellung der Verbindungen der Formel (I) gemäß Anspruch 1, für die R einen CH-R₆-Rest darstellt, in dem R₆ einen -NR₁₄R₁₅-Rest darstellt, R₁₄ ein Wasserstoffatom darstellt, R₁₅ einen -COR₂₂-Rest darstellt und R₂₂ einen Alkylrest darstellt, dadurch gekennzeichnet, daß man ein Reduktionsmittel mit einer entsprechenden Verbindung der Formel (I), für die R einen C=R₇-Rest darstellt und R₇ einen NOH-Rest darstellt, umsetzt, anschließend mit einem Anhydrid (RqCO)₂O, für das Rq einen Alkylrest darstellt, behandelt, das Produkt isoliert und es gegebenenfalls in ein Salz überführt.

8. Verfahren zur Herstellung der Verbindungen der Formel (I) gemäß Anspruch 1, für die R₁ und gegebenenfalls R₂ einen -NH-CO-NR₁₁R₁₂-Rest darstellen und R₁₂ ein Wasserstoffatom darstellt, dadurch gekennzeichnet, daß man eine entsprechende Verbindung der Formel (I), für die R₁ einen Aminorest darstellt, mit einem Derivat Rx=C=N-Ry, in dem Rx ein Sauerstoffatom darstellt und Ry einen Phenylrest darstellt, umsetzt, das Produkt isoliert und es gegebenenfalls in ein Salz überführt.

9. Medikamente, die als Wirkstoff wenigstens eine Verbindung der Formel (I) gemäß einem der Ansprüche 1 oder 2 oder ein pharmazeutisch annehmbares Salz einer solchen Verbindung enthalten.
